# EUROPÄISCHE PATENTANMELDUNG

(11) **EP 1 054 061 A1**
(43) Veröffentlichungstag der Anmeldung: **22.11.2000**
(21) Anmeldenummer: 99109786.6
(22) Anmeldetag: 18.05.1999
(51) Int. Cl.: C12N 15/31, C07K 14/315, G01N 27/447

(54) **Standardprotein, das mindestens eine Antikörperdomäne des Protein G aus Streptococcus enthält**

(71) Anmelder: Uhlenküken, Jochen, 48151 Münster (DE); Schmidt, Gerd, 48151 Münster (DE); Lansing, Manfred, 48151 Münster (DE)
(72) Erfinder: Uhlenküken, Jochen, 48151 Münster (DE); Schmidt, Gerd, 48151 Münster (DE); Lansing, Manfred, 48151 Münster (DE)
(74) Vertreter: Meyers, Hans-Wilhelm, Dr.Dipl.-Chem.

(57) **Zusammenfassung**

Standardprotein mit mindestens einer Antikörperbindungsdomäne aus Protein G, die mindestens einen nicht-konservativen Austausch einer Aminosäure aufweist.

## Beschreibung

Die Erfindung betrifft ein Standardprotein sowie Mischungen von Standardproteinen, einen Expressionsvektor zur Expression des Standardproteins und ein Verfahren zur Herstellung des Standardproteins.

In der Analytik von Proteinen besitzt insbesondere die Bestimmung des Molekulargewichts und des isoelektrischen Punktes einen großen Stellenwert. Die Analyse erfolgt allgemein durch die Bestimmung der Wanderungsgeschwindigkeit oder - strecke des Proteins unter chromatographischen oder elektrophoretischen Bedingungen. Da diese Verfahren keine Absolutwerte geben und eine große Schwankungsbreite aufweisen, ist es erforderlich, die Eigenschaften im Vergleich zu einem Standardprotein zu bestimmen, dessen Eigenschaften bekannt sind.

Eine übliche Methode zur Bestimmung der Eigenschaften eines Proteins in einer Mischung von Proteinen ist z.B. der Western-Blot (Bumette, W.N.: "Western Blotting": Electrophoretic transfer of proteins from sodium dodecyl sulfate polyacrylamid gels to unmodified nitrocellulose and radiographic detection with antibody and radiolabelled protein, A. Anal. Biochem. 112 (1981) 195-203). Dazu wird ein Proteingemisch z.B. durch Polyacrylamidgelelektrophorese (Laemmli, U.K. "Cleavage of Structural Proteins During the Assembly of the Head of the Bacteriophage T4", Nature 227 (1970) 680-685) aufgetrennt. Nach einer eventuellen Färbung wird das Gel auf eine proteinbindende Membran (z.B. aus Nitrocellulose oder Polyvinyldifluorid (PVDF)) gelegt und in einer Blotkammer elektroeluiert, wobei die Proteine aus dem Gel heraus auf die Membran wandern. Restliche freie Proteinbindungsstellen auf der Membran werden mit einem Blockierungsreagenz, das z.B. Albumin enthalten kann, abgesättigt.

Der Nachweis des zu analysierenden Proteins erfolgt durch Inkubation mit einem gegen dieses Protein gerichteten sogenannten primären Antikörper, der mit möglichst hoher Affinität und Spezifität an das Protein bindet. In einem zweiten Schritt wird mit einem gegen den Fc-Teil des primären Antikörpers gerichteten sogenannten sekundären Antikörper inkubiert, an dem ein Marker, üblicherweise ein Enzym, konjugiert ist. Im Fall des Enzyms ist dieses in der Lage, in einem weiteren Schritt ein zugesetztes Substrat in ein farbiges, unlösliches Produkt umzusetzen und damit im Endeffekt die Position des entsprechenden Proteins spezifisch zu bestimmen.

Problematisch ist hierbei jedoch, daß durch dieses Verfahren die Positionen der Standardproteine nicht mitgefärbt werden. Eine allgemeine Anfärbung mit einem Proteinfärbereagenz ist nicht möglich, da dadurch auch die Proteine der Proteinmischung des zu untersuchenden Proteins angefärbt würden.

Nicht unüblich ist es daher, die Membran mit den geblotteten Standardproteinen von der restlichen Membran abzuschneiden und nur den Standard einem Färbeprozeß zu unterwerfen. Durch den Einsatz von organischen Lösungsmitteln in der Färbelösung kommt es jedoch häufig zu einer Dehnung oder Schrumpfung der Membran, so daß eine hinreichend exakte Zuordnung der Eigenschaft des Standardproteins und der Eigenschaft des zu untersuchenden Proteins unmöglich wird.

Eine andere Methode, die eine Aufteilung der Membran für eine getrennte Entwicklung der Standardproteine und der restlichen Membran nicht mehr benötigt, ist die Verwendung vorgefärbter Standards. Nachteilig sind aber diffuse unscharfe Banden, die sich nach dem Blotting der Standardproteine auf die Membran ergeben. Weiterhin nachteilig ist eine Variabilität zwischen verschiedenen Produktionschargen, die eine entsprechende Kalibrierung der Chargen notwendig macht. Im Ergebnis werden die vorgefärbten Standards als Blotkontrolle und zu groben Größenabschätzung eingesetzt.

Eine weitere Methode besteht darin, die Standardproteine zu markieren und über diese Markierung zu detektieren. Handelsüblich sind z.B. mit Biotin markierte Standardproteine, die mittels des Biotin/Streptavidin-Systems nachgewiesen werden können. Andere Standardproteine werden über molekulargenetische Methoden mit kurzen Peptidsequenzen, den sogenannten tags, versehen. Der Nachweis gelingt dann mittels spezifischer Antikörpern gegen die tags. Nachteilig ist aber, daß das zu untersuchende Protein ebenfalls biotinyliert oder mit einem tag versehen sein muß. Für viele analytische und diagnostische Zwecke, die verschiedenste Antikörpern benötigen oder die nicht charakterisierte Probengemische verwenden sind diese Systeme daher nicht brauchbar.

Verschiedene Detektionsverfahren werden z.B. von Timmons und Dunbar (1990) beschrieben (Timmons, T.M. und Dunbar B.S.: Protein blotting and immunodetection. In: Murray P. Deutscher (Hrsg.): Methods in Enzymology. 182, 1990, 679-688)

Davies et al. (WO 86/06383) beschreiben die Nutzung von Antikörperfragmenten und Polymere daraus, die als Molekulargewichtsstandards genutzt werden können. Der Nachweis der beschriebenen Proteine wird im Westernblot über spezifische Antikörper geführt, die gegen die Antikörperfragmente gerichtet sind. Nachteilig bei diesem Verfahren sind die ungeraden Molekulargewichte, die sich aus der Fragmentierung der Antikörper ergeben, die Glykosilierung einiger Antikörperfragmente und die Notwendigkeit, die Detektion des Molekulargewichtsstandards und der zu untersuchenden Probe mittels Antikörper durchzuführen, die aus der gleichen Spezie gewonnen werden müssen. Ist Letzteres nicht der Fall müssen unterschiedliche Nachweissysteme für den Standard und Probe benutzt werden, die aus unterschiedlichen primären und sekundären Antikörpern bestehen.

Eine weitergehende Entwicklung stellt die Nutzung der Immunoglobulinbindenden Domänen von Protein A und Polymere daraus dar (EP-A-0 550 771).

Lindbladh et al. und Zueco und Boyd beschreiben Fusionsproteine von Protein A als Molekulargewichtsstandards (Lindbladh et al.: "Standard calibration proteins for westernbotting obtained by genetically prepared protein A conjugates" Analytical Biochemistry, 197 (1991) 187-190; Zueco und Boyd: "Protein A fusion proteins as molecular weight markers for use in immunoblotting" Analytical Biochemistry, 207 (1992) 348-349)

Nachteilig ist, daß sich Protein A in der SDS-PAGE nicht wie erwartet verhält; es besitzt eine größere Mobilität, als sich aus der berechneten Aminosäuresequenz ergeben würde. Besonders bei kleineren Molekulargewichten (< etwa 20 kDa) führt dieser Effekt zu einer Unsicherheit in der Molekulargewichtsbestimmung. Weiterhin nachteilig ist, daß Protein A weniger Immunoglobulintypen erkennt als Protein G und somit das Spektrum der zu verwendenden Antikörper eingeschränkt ist.

Weiterhin nachteilig ist, daß Protein A nicht an das Fab-Fragment der Antikörper bindet. Fab-Fragmente sind von zunehmendem Interesse, da sie leicht in größeren Mengen als rekombinante Proteine in Escherichia coli hergestellt werden können. (Winter und Milstein: " Man-made antibodies" Nature, 349 (1991) 293-299; Pack und Plückthun: "Miniantibodies; Use of amphipathic helices to produce functional, flexibly linked dimeric Fv fragments with high avidity in Escherichia coli. Biochemistry 31 (1992) 1579-1584).

Wie Protein A verhält sich aber auch Protein G in der SDS-PAGE nicht entsprechend seines Molekulargewichtes. Protein G besitzt eine geringere Mobilität, als sie sich aus der berechneten Aminosäuresequenz ergeben würde (Guss et al, EMBO J 5, 567-575, 1986). So haben Goward und Mitarbeiter (Goward, CR, Murphy, JP, Atkinson, T, Barstow, DA.: Expression and purification of a truncated recombinant streptococcal protein G. Biochemical Journal 267: 171-177, 1990) ein verkürztes rekombinantes Protein G in E. coli exprimiert, das ein apparentes Molekulargewicht von 35 kDa zeigt, obwohl entsprechend der Aminosäuresequenz ein Protein von 20 kDa zu erwarten war.

Die Antikörperbindungsdomäne von Protein G besteht aus vier antiparallelen b-Faltblättern, die schalenförmig angeordnet sind und über die sich diagonal eine Helix spannt. Nach außen schirmen geladene Aminosäurereste einen inneren hydrophoben Kern ab. Diese abgekapselte Struktur wird für die Stabilität der Domäne und für das anormale Laufverhalten verantwortlich gemacht, wobei vermutet wird, das nicht genügend SDS an das Molekül angelagert werden kann. (Gronenborn et al.: A novel, highly stable fold of the immunoglobulin binding domain of streptococcal protein G. Science, 253: 657-661, 1991; Goward et al. Biochemical Journal 267: 171-177, 1990)

In Mutationsexperimenten mit dem Ziel den Einfluß von Aminosäureaustausche auf die Stabilität der Antikörperbindungsdomäne des Proteins G zu bestimmen wurden gezielte und zufällige Mutationen untersucht (Smith, C K, Withka, J M, Regan, L: A thermodynamic scale for the b-sheet forming tendencies of the amino acids. Biochemistry, 33: 5510-5517, 1994; O'Neil, K, Hoess, R H, Raleigh, D P, DeGrado, W F: Thermodynamic genetics of the folding of the B immunoglobulin-binding domain from streptococcal protein G. Proteins: Structure, Function and Genetics, 21: 11-21, 1995). Diese Mutatiosexperimente zeigten, daß die Antikörperbindungsdomäne von Protein G stark destabilisiert werden kann, ohne das die Antikörperbindung verloren geht. Die erzeugten Mutationen wurden aber nicht auf ihr Laufverhalten in der SDS-PAGE untersucht.

Das technische Problem, das der vorliegenden Erfindung zugrunde liegt, ist es daher, Standardproteine bereitzustellen, die in einfacher Weise eine selektive Anfärbung der Standardproteine im Westernblot erlauben und darüber hinaus kompatibel zu einer größtmöglichen Vielfalt von laborüblichen chromatographischen oder elektro- phoretischen Trenn- und Detektionsverfahren sind und deren apparentes und tatsächliches Molekulargewicht möglichst übereinstimmen.

Das Problem wird gelöst durch ein Standardprotein mit den Merkmalen des Anspruchs 1.

Das erfindungsgemäße Standardprotein weist mindestes eine Antikörperbindungsdomaine aus Protein G mit mindestens einem nicht kongerativen Austausch einer Aminosäure auf. Dadurch können z.B. im Western-Blot das zu analysierende Protein und das Standardprotein bzw. die Standardproteine durch Zugabe eines enzymkonjugierten, sekundären Antikörpers in einem gemeinsamen Schritt gleichzeitig angefärbt werden.

"Antikörperverbindungsdomaine" ist der Bereich des Protein G, der für die Bindung an den Antikörper verantwortlich ist. Protein G ist beispielsweise beschrieben in Fahnestock, SR, Alexander, P, Nagle, J, Filpula, D: Gene for an immunoglobulin-binding protein from a group G streptococcus. J Bacteriol 167, 870 ― 880, 1986;

"Konservativer Austausch" ist ein Austausch innerhalb der Gruppen der neutralen, schwach hydrophoben Aminosäuren (P, A, G, S, T), der hydrophilen sauren Aminosäuren (Q, N, E, D, B, Z), der hydrophilen basischen (H, K, R) und der hydrophoben (L, I, V, M) und der hydrophoben aromatischen Aminosäure (F, Y, W). "Nicht-konservativer Austausch" bedeutet ein Austausch zwischen den eben definierten Gruppen, eine Insertion oder eine Deletion.

"Konstante Regionen" eines Antikörpers bezeichnen die konstanten Anteile der leichten und/oder schweren Ketten der Antikörper. Im Falle des Immunglobulin G sind dies die konstante Region der H-Kette (C_{H}) mit den Abschnitten C_{H}1, C_{H}2, C_{H}3 sowie die konstanten Regionen der L-Kette (C_{L}). Bevorzugt handelt es sich um Bereiche aus der Fc-Einheit des Antikörpers.

Unter "denaturierenden Bedingungen durchgeführten elektrophoretischen Trennung" wird eine Bedingung verstanden, die bei elektrophoretischen Trennungen üblicherweise eingehalten wird, wie Behandlungen mit Detergenzien z.B. SDS, reduzierenden Milieu, Harnstoff etc. Insbesondere wird darunter ein Erhitzen auf 95°C für 5 Minuten in Gegenwart von Solubilizer wie in Beispiel 2 verstanden.

Unter "funktional" wird verstanden, daß nach Durchlaufen der denaturierenden Bedingung unter sonst üblichen Färbebedingungen eine Erkennung der Bindungsstelle des Standardproteins möglich ist.

Die erfindungsgemäße Standardproteine enthalten mindestens einen nicht-konservativen Austausch. Bevorzugt enthalten sie 2 bis 7 nicht-konservative Austausche, am meisten bevorzugt 3, 4 oder 5 Austausche. Es sollten nicht mehr als 10% der Aminosäuren ausgetauscht werden, da dies im allgemeinen die Bindungsfähigkeit des Protein G zu stark herabsetzt.

Insbesondere vorteilhaft ist es, wenn die Bindungsstelle des erfindungsgemäßen Standardproteins in der Lage ist, einen stabilen Komplex mit dem Antikörper zu bilden, um den Nachweis möglichst lokalisiert durchzuführen.

Bevorzugt ist, daß es sich bei dem Standardprotein um ein rekombinantes Protein handelt.

Es ist insbesondere vorteilhaft, wenn das Standardprotein ein rekombinantes Protein ist, das aus ein bis mehreren Molekulargewichtsdomänen 1 besteht, das mit einem oder mehreren Bindungsdomänen 2 ein rekombinantes Fusionsprotein bildet. Durch die mehrmalige Wiederholung dieser Module können sowohl die Proteineigenschaften des Standards als auch die Bindungsstellen in besonders einfacher Weise vielfach kombiniert werden.

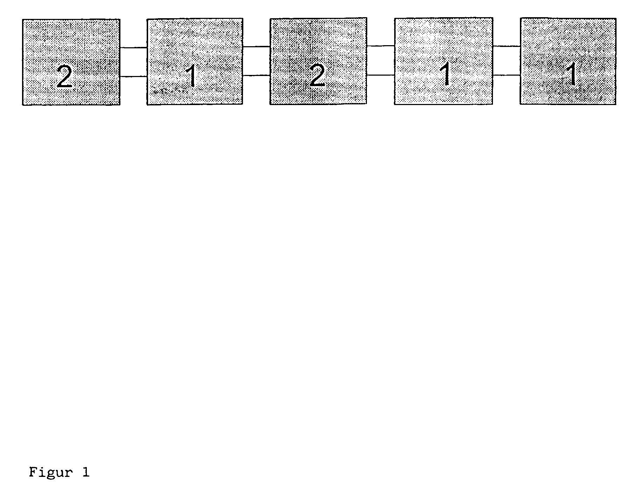

Figur 1 zeigt schematisch eine bevorzugte Ausführungsform.

Das erfindungsgemäße Standardprotein kann eine oder mehrere Bindungsstellen für eine konstante Region eines Antikörpers und auch mehrere verschiedene Bindungsstellen im gleichen Protein aufweisen.

Es ist vorteilhaft Bindungsdomänen für die konstanten Regionen von Antikörpern zu verwenden, die ein weites Spektrum von Antikörpern verschiedener Spezies erkennen, wie dies bei Protein G der Fall ist. Gene für Protein G aus verschiedenen Streptokokken-Isolaten wurden beschrieben (Guss, B, Eliasson, M, Olsson, A, Uhlen, M, Frej, A-K, Jörnvall, H, Flock, J-I, Lindberg, M: Structure of the IgG-binding region of streptococcal protein G. EMBO J 5, 567-575, 1986; Fahnestock, S, Alexander, P, Nagle, J, Filipula, D: Gene for an immunoglobulin-binding protein from a group G streptococcus. J Bacteriol 167: 870-880 1986).

Bevorzugte Mutationen im Sinne dieser Erfindung sind die der Austausch des Threonins53 gegen Arginin (T53R) und des Glutaminsäure56 gegen Threonin (E56T) im vierten b-Faltblatt, des Alanins26 gegen Histidin (A26H) und des Glutaminsäure27 gegen Lysin (E27K). Es liegt aber auch nahe, daß andere Mutationen im Sinne der Erfindung zu einer Antikörperbindungsdomäne verwendet werden können, die dazu führen, daß die Antikörperbindungsdomäne des Proteins G sich im SDS-PAGE so verhält, wie es das vorausberechnete Molekulargewicht erwarten läßt.

Bevorzugt sind Austausche zwischen den Gruppen der neutralen schwach hydrophoben, der hydrophilen sauren und der Gruppe der hydrophilen basischen Aminosäuren. Zusätzlich können auch einige weitere konservative Austausche innerhalb der Gruppen vorhanden sein, solange sie die Bindungsfähigkeit der Antikörperbindungsdomäne des Protein G nicht zu stark verringern. Bevorzugt ist der Austausch von höchstens 10% der Aminosäuren.

Vorteilhafterweise können die DNA-Sequenzen für die so gewonnenen Bindungsdomänen für die konstanten Antikörperanteile über PCR amplifiziert und gegebenenfalls modifiziert werden, so daß sie mit einer Molekulargewichtsdomäne verknüpft werden können, um ein Standardprotein herzustellen.

Weiterhin können in dem Standardprotein zusätzliche Mutationen vorhanden sein, die eine Derivatisierung ermöglichen. So erlauben beispielsweise eingeführte Cystein-Reste die Verknüpfung mit Enzymen, die für die Detektion von Antikörpern eingesetzt werden wie alkalische Phosphatase oder Meerrettichperoxidase über übliche Crosslinker. Für die Koppelung von Farbstoffen, z.B. Azofarbstoffe, eignen sich Histidin- oder Tyrosinreste. Weiterhin eignen sich gezielt eingeführte Aminosäurereste ebenfalls für eine radioaktive Markierung wie z.B. Tyrosinreste für die Markierung mit radioaktivem Iod.

Darüber hinaus ist eine radioaktive Markierung der erfindungsgemäßen Standardproteine auch über den Einbau von radioaktiv markierten Aminosäuren während der Expression des Standardproteins möglich.

Die Bindungsstellen des erfindungsgemäßen Standardproteins liegen vorteilhaft am N- oder C-Terminus, da dadurch ein freier Zugang des Bindungspartners erleichtert wird. Da die Bindung aber häufig nach einer Denaturierung erfolgt, z.B. nach einer Polyacrylamidgelelektrophorese, können die Bindungsstellen auch über das gesamte Protein verteilt sein, soweit zumindest ein Teil davon für die zu bindenden Enzyme erreichbar ist.

Insbesondere vorteilhaft ist es, wenn das Standardprotein nicht posttranslational modifiziert ist, insbesondere nicht glykosyliert wird. Posttranslationale Modifikationen können zu einer sogenannten Mikroheterogenität führen, wodurch Proteine mit der gleichen Aminosäuresequenz durch die Kopplung weiterer Substanzen eine Molekulargewichtsbandbreite aufweisen, die sich nachteilig auf die Schärfe der durch die chromato- graphische oder elektrophoretische Trennung erhaltenen Banden oder Fraktionen auswirkt. Nicht posttranslational modifizierte Proteine zeigen demgegenüber eine bessere, weil schärfere Trennung.

Zumeist wird es sinnvoll sein, eine Mischung der Standardproteine einzusetzen, um durch Interpolation die Eigenschaften des zu untersuchenden Proteins möglichst exakt bestimmen zu können. Die erfindungsgemäßen Standardproteine in der Mischung können sich zum Beispiel in ihrem Molekulargewicht unter- scheiden.

Da die Wanderungsgeschwindigkeit eines Proteins in einer Polyacrylamidgelelektrophorese unter denaturierenden Bedingungen (Zusatz von Natriumdodecylsulfat (SDS)) in einem logarithmischen Zusammenhang mit dem Molekulargewicht des Proteins steht, ist es besonders vorteilhaft, wenn sich die Molekulargewichte der Standardproteine um den Faktor 2 unterscheiden, da dadurch ein in etwa gleicher Abstand der Proteinbanden im Gel erreicht wird.

Aufgrund des Trennbereichs der Polyacrylamidgelelektrophorese werden insbesondere Mischungen bevorzugt, die Standardproteine mit einem Molekulargewicht von 5, 10 bis 120 kDa in 10er Abständen und 150 - 500 kDa in 50er Abständen enthalten. Auch Mischungen mit einem engeren Molekulargewichtsabstand können - je nach Trennproblem - vorteilhaft sein. Einzelne Standardproteine können in deutlich höherer Menge als andere Standardproteine eingesetzt werden, um die Zuordnung der Banden zu erleichtern.

Alternativ dazu können sich die Standardproteine jedoch auch in ihrem isoelektrischen Punkt (pI) unterscheiden.

Isoelektrische Fokusierung (IEF) erfolgt in einem Gel, das einen pH-Gradienten aufweist (Bollag, D.M., Edelstein, S.J. (eds.): "Protein methods" Wiley-Liss, New-York, NY, 1991 und Cooper, T.G. (Hrsg.): "Biochemische Arbeitsmethoden" Walter de Gruyter, Berlin, BRD, 1981). Bei der IEF wandern die Proteine entsprechend ihrer Eigenladung in einem pH-Gradienten, wobei dieser pH-Gradient je nach Technik entweder schon im Gel vorliegt (Immobiline-Technik) oder sich erst beim Anlegen einer Spannung entwickelt (Carrier-Ampholyte-Technik). Die Proteine wandern solange, bis ihr pI dem pH des Gels entspricht. Die native IEF benötigt im Gegensatz zu der weit häufiger verwendeten denaturierenden Technik keinen Harnstoffzusatz. Sinn des Harnstoffeinsatzes ist die Vermeidung von Aggregationen; durch auftretende Aggregationen erreicht die native nicht die Auflösung der denaturierenden IEF.

Für einen linearen pH-Gradienten ist es besonders vorteilhaft, wenn sich die Standardproteine jeweils um den gleichen Wert unterscheiden, wobei je nach Auflösung des Gels bzw. der Breite des Gradienten der Unterschied der isoelektrischen Punkte zwischen den Proteinen 0,5 oder 1 betragen sollte.

Vorzugsweise sollte die Mischung einen pH-Bereich von 3 bis 11 abdecken. Für eine exakte Bestimmung des isoelektrischen Punktes kann es jedoch auch vorteilhaft sein, nur Teilbereiche des pH-Spektrums abzudecken.

Für mehrdimensionale Gele kann es vorteilhaft sein, wenn sich die Proteine in mehr als einer Eigenschaft unterscheiden.

Neben der Möglichkeit, vorbereitete Mischungen der Standardproteine bereitzustellen, kann es auch vorteilhaft sein, dem Anwender eine Zusammenstellung mehrerer Standardproteine in getrennten Gefäßen anzubieten, aus denen er, unter Berücksichtigung des betreffenden analytischen Problems, eine individuelle Mischung von Standardproteinen zusammenstellt.

Bevorzugterweise werden die Standardproteine durch Expression eines Expressionsvektor in einem geeigneten Expressionssystem hergestellt. Der Expressionsvektor kodiert unter anderem für das Expressionsprotein, das sich vorteilhafterweise aus mindestens einem, sich jedoch bevorzugt wiederholenden Molekulargewichtsdomänen und ebenso eine oder mehreren Bindungsdomänen aufweist. Durch an sich bekannte molekularbiologische Techniken kann somit aus einem Stammvektor durch Vervielfachung der Nukleotidsequenzen für die Proteindomänen ein Expressionsvektor für ein gewünschtes Standardprotein erhalten werden.

Während die Verwendung der erfindungsgemäßen Standardproteine bzw. ihrer Mischung insbesondere im Western-Blot vorteilhaft ist, eignen sie sich ebenso gut für andere Analysenmethoden.

Sie können nach Einsatz in einer Polyacrylamidgelelektrophorese auch mit Hilfe üblicher Färbemethode, z.B. Coomassie-Blau oder Silberfärbung angefärbt werden. Auch hierbei sind die exakt definierten Eigenschaften vorteilhaft.

Soweit die erfindungsgemäßen Standardproteine aus Modulen aufgebaut sind, ist davon auszugehen, daß die Anfärbung der erfindungsgemäßen Standardproteine relativ gleichmäßig erfolgt.

Aufgrund ihrer exakt definierten Eigenschaften eignen sich die erfindungsgemäßen Standardproteine auch für weitere analytische Verfahren z.B. Kapillar-Elektrophorese, Gelpermeationschromatographie und Massenspektroskopie. Weiterhin können die erfindungsgemäßen Standardprotein für enzymgekoppelte Detektionsverfahren von Antikörpern oder immobilisiert als stationäre Phase zur Isolierung von Antikörpern genutzt werden.

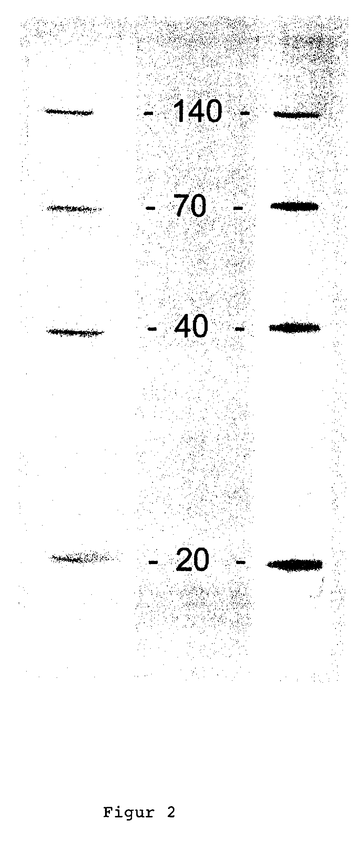

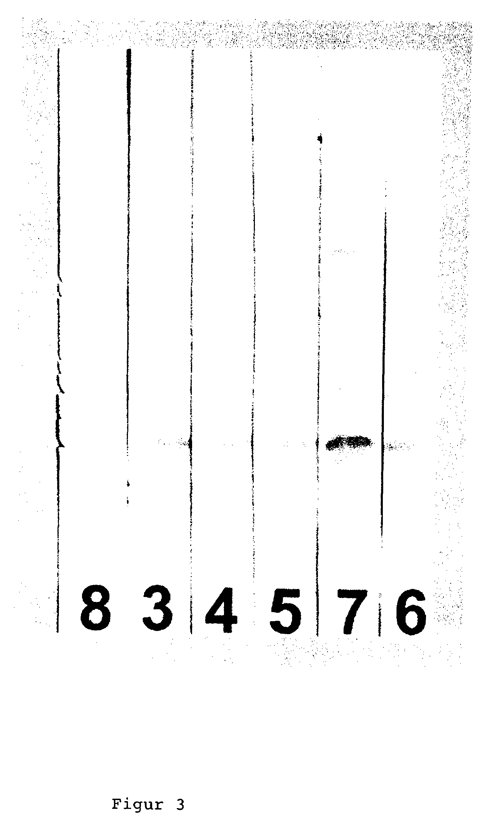

Figur 2 zeigt Banden der erfindungsgemäßen Proteine mit den Größen 20, 40, 70 und 140 kD im SDS-Polyacrylamidgel nach Laemmli (links) und im Western-Blot (rechts). Die hier dargestellten Proteine sind vor der elektrophoretischen Trennung mittels Gelfiltration über eine Superdex 200 HR Säule unter Einsatz einer FPLC Anlage gereinigt worden.

Die Herstellung und Verwendung der erfindungsgemäßen Standardproteine bzw. ihrer Mischung wird durch die folgenden Beispiele näher erläutert.

### Beispiel 1:

### Herstellung eines Fusionsproteins mit einer Antikörperbindungsdomäne

Die DNA-Manipulationen wurden entsprechend Sambrook J, Fritsch EF, Maniatis, T. Molecular Cloning: A Laboratory Manual. Cold Spring Harbor Laboratory Press, Cold Spring Harbor, NY (1989) durchgeführt. Grundlegende Ausführungen zur PCR finden sich bei McPherson, M J, Quirke P, Taylor, G R (Eds.): PCR: A practical Approach. IRL Press, Oxford, England (1991).

Über PCR wird vom Gen des Proteins G ein DNA-Fragment amplifiziert, das für eine Antikörperbindungsdomäne codiert. Das Fragment wird in einen Expressionsvektor ligiert, der für ein Fusionsprotein mit einer Antikörperbindungsdomäne codiert.

Die Plasmid-DNA des Expressionsvektors wird als Ausgangsmaterial für die Mutationen im Sinne der Erfindung genutzt.

In den mit den entsprechenden Mutationen versehenen Expressionsvektor können DNA-Fragmente ligiert werden, die für verschiedene Molekulargewichtsdomänen codieren. Molekulargewichtsdomänen können z.B. für 10, 20, 30, 40 kDa etc codieren.

### Herstellung eines Expressionsvektors:

Abgeleitet vom Plasmid pAX4a (Markmeyer, P, Rühlmann, A, Englisch, U, Cramer, F: The pAX plasmids: new gene fusion vectors for sequencing, mutagenesis and expression of proteins in Escherichia coli. Gene 93, 129-134,1990) wurde mittels PCR ein Plasmid (p20) hergestellt, das für ein 20 kDa Protein codiert Nach Transformation von kompetenten Escherichia coli XL-1 Zellen (Stratagene GmbH, Heidelberg, Deutschland), mittels Elektroporation wurden die Zellen auf LB/Ampicillin (100 µg/ml) Agarplatten gebracht. Es wurde ein XL-1 Klon gesucht, der das 20 kDa Protein nach Induktion mit IPTG exprimiert (s.a. unten Expression). Die Plasmid DNA des entsprechenden Klons (XL-1/p20) wurde isoliert um eine Antikörper- bindungsdomäne und/oder die Molekulargewichtsdomänen aufzunehmen.

### Herstellung der Antikörperbindungsdomäne:

Der Streptokokkenstamm G148 (Stammsammlung der Universität Göteborg, Guldhedsg. 10, Göteborg, Schweden) wurde auf Todd-Hewitt Agar ausgestrichen und bei 30°C bebrütet. Ein Abstrich der Bakterienkultur wurde in 1% Tween-20 für 15 min gekocht und mit einem 5 µl Aliquot und den Primern GA (cgaattcaggt tactgaaaaacc-3') und GB (aacaaggaattcaggttgtcacggc-3') mittels PCR die Antikörperbindungsstelle amplifiziert. Das PCR-Produkt wurde in pGEM-T (Promega GmbH, Mannheim, Deutschland) ligiert und XL-1 Zellen transformiert. Bakterienklone, die die DNA für die Antikörperbindungsstelle enthielten wurden gesucht und die Plasmid-DNA isoliert. Mit der Restriktionsendonuklease EcoRI wurde die DNA für die Antikörperbindungsstelle herausgeschnitten und in das entsprechend vorbereitete p20-Plasmid-DNA ligiert. Nach Transformation von kompetenten Escherichia coli XL-1 Zellen wurde ein Klon (XL-1/p20) gesucht, der ein Fusionsprotein mit einer Antikörperbindungsstelle exprimiert. Von diesem Klon wurde die Plasmid-DNA isoliert. Das Fusionsprotein hat ein errechnetes Molekulargewicht von 30 kDa, besitzt aber nach SDS-PAGE ein apparentes Molekulargewicht von etwa 33,5 kDa.

### Mutationen der Antikörperbindungsdomäne:

Von der Antikörperbindungsdomäne wurde das interne ClaI-Fragment in den entsprechend vorbereiteten Expressionsvektor gebracht und das exprimierte Fusionsprotein untersucht. Der Vektor (p1433) codiert für ein Fusionsprotein mit 27,8 kDa, das exprimierte Protein besitzt aber nach SDS-PAGE ein apparentes Molekulargewicht von 30,1 kDa. Von dem Expressionsvektor p1433 wurden verschiedene Mutationen mittels PCR hergestellt (s.u.). Die PCR-Primer wurden dabei so ausgewählt, das am 5'-Ende eines Primers das Triplett für die auszutauschenden Aminosäure gesetzt wird.

### Destabilisierung der Antikörperbindungsdomäne:

Entsprechend der Mutationsversuche von Smith et al. (1994) und 0'Neil et al. (1995) wurden Aminosäureaustausche durchgeführt, um die Antikörperbindungsdomäne zu destabilisieren. Dazu wurde das Threonin53 gegen Proline (T53P) ausgetauscht, bzw. deletiert (DT53) und das Alanine 26 gegen Proline ausgetauscht (A26P). Die von den entsprechenden Klonen exprimierten Proteine wurde auf ihr Laufverhalten in der SDS-PAGE und ihre Antikörperbindungseigenschaft mittels Westernblot hin untersucht . Die Proteine besitzen nach SDS-PAGE ein deutlich höheres apparentes Molekulargewicht als die entsprechenden nicht mutierten Proteine.

### Mutationen im hydrophoben Kern der Antikörperbindungsdomäne:

Über PCR wurde mit entsprechenden Primern Threonin53 gegen Arginin (T53R) ausgetauscht und das Glutaminsäure56 gegen Threonin (E56T) ausgetauscht. Das Alanin 26 wurde gegen Histidin ausgetauscht (A26H) und das Glutaminsäure 27 gegen Lysin (E27K). Weiterhin wurden mittels PCR die N- und C-terminalen Proline entfernt. Die von den entsprechenden Klonen exprimierten Proteine wurde auf ihr Laufverhalten in der SDS-PAGE und ihre Antikörperbindungseigenschaft mittels Westernblot hin untersucht.

Es wurden diejenigen Klone ausgewählt, die Protein exprimieren, deren apparentes Molekulargwicht mit dem errechneten übereinstimmt. Die mutierten Antikörperbindungsdomäne wurde über PCR als 10 kDa Antikörperbindungsdomäne amplifiziert und in den Expressionvektor p20 zur Expression eine 30 kDa Proteins benutzt.

### Herstellung der Molekulargewichtsdomäne:

Ausgehend vom lacZ-Gen des Plasmids pUR290 (Rüther, U, Müller-Hill, B: Easy identification of cDNA clones. EMBO J 2, 1791-1794, 1983) wurde eine Molekulargewichtsdomäne von 10 kDa mittels PCR und den Primern 10kDA (ccatgggaagctggctggagtg-3')und 10kDB (ccatggcgcgcccgttgcacca-3') hergestellt, das PCR- Produkt in pCR2.1 (Invitrogen BV, 9351 NV Leek, Niederlande) ligiert und XL-1 Zellen transformiert. XL-1 Klone, die die DNA für die 10 kDa Molekulargewichtsdomäne enthielten, wurden gesucht und die Plasmid-DNA isoliert. Das DNA-Fragment für die 10 kDa Domäne wurde mit der Restriktionsendonuklease NcoI herausgeschnitten und nach entsprechender Vorbereitung in die NcoI Schnittstelle des p20 Plasmids ligiert. Nach Transformation wurde ein Klon gesucht, der ein 30 kDa Protein exprimiert (XL-1/p20).

### Expression:

Einzelne Klone wurden von der LB-Ampicillin (100 µg/µl Ampicillin) Agarplatte gepickt und über Nacht in 200 µl LB Medium mit Ampicillin (1mM f.c.) bei 37°C im Schüttelinkubator inkubiert. Von der Zellsuspension wurden 20 µl in 200 µl LB Medium mit Ampicillin (1mM f.c.) und Isopropyl-β-D-1-thiogalactopyranosid (IPTG, 1mM f.c.) überführt und für 6 Stunden bei 37°C im Schüttelinkubator inkubiert.

### Beispiele 2 bis 9

Anwendungen von Standardproteinen mit einer Bindungsstelle zu den konstanten Regionen der leichten und/oder schweren Kette von Antikörpern

### Beispiel 2: Gelelektrophorese

Nach der Expression wurde die Zellsuspension mit 19.000 x g 3 min. zentrifugiert. Der Überstand wurde vorsichtig abgenommen und verworfen, das Zellpellet wurde in 100 µl Solubiliser (10% Glycerin, 2% SDS, 5% β-Mercaptomethanol, 50 mM TRIS/HCl (pH 6,8), Bromphenolblau) gelöst und in einem Heizblock 5 Minuten auf 95°C erhitzt.

Danach wurde nochmals 3 min. mit 19.000 x g zentrifugiert und vom Überstand jeweils 5 µl auf ein 12,5 % SDS - Polyacrylamidgel (0,75 mm, 7 x 8 cm)gegeben.

Die Polyacrylamidgelelektrophorese (PAGE) nach Laemmli (Nature, 227, 680, 1970) erfolgte für ca. 50 min. mit einer konstanten Spannung von 200 V.

Nach der PAGE wurde das Gel in einer Coomassie-blue Lösung (0,1% Coomassie Blue, 40% Methanol, 60% Eisessig) 60 min gefärbt, und anschließend in einer Entfärbelösung (40% Methanol, 60% Eisessig) soweit entfärbt, daß sich die einzelnen Proteinbanden klar vor dem Hintergrund abhoben.

### Western-Blot und Immundetektion:

Die Proteine eines zweiten Gels wurde nach der PAGE in einer handelsüblichen Western-Blot Apparatur für 2 Stunden bei einem konstanten Strom von 210 mA auf eine Nitrocellulosemembran elektrotransferiert. Die Membran wurde dann zunächst für 30 min. mit 10 ml einer 3% Magermilch Blocklösung inkubiert. Nach einem dreimaligen Waschschritt mit je 10 ml TBST Puffer (50 mM TRIS/HCl, 150 mM NaCl, 0,1% Tween 20, pH 7,5) erfolgte eine 30 min. Inkubation in 10 ml der primären Antikörperlösung (Kaninchenserum 1:3.000 in Blocklösung). Nach abermaligen Waschen wurde die Membran 30 min. mit 10 ml des mit alkalische Phosphatase gekoppelten sekundärem Antikörpers (Anti-Rabbit-IgG 1:10.000 in 3% Blocklösung) inkubiert und danach nochmals gewaschen. Nach dem letzten Waschschritt wurde die Membran kurz mit dem Substratpuffer (50 mM TRIS/HCl, 150 mM NaCl, 5 mM MgCl2, pH 9,5) gespült. Unmittelbar danach erfolgte die Entwicklung der Membran in 10 ml Substratlösung mit Nitro-Blue-Tetrazolium (NBT, 330 µg/ml) und 5-Bromo-4-Chloro-3-Indoyl Phosphat (BCIP, 165 µg/ml). Die Entwicklung erfolgte für 5 min. und wurde dann durch Spülen mit Wasser gestoppt.

### Beispiel 3

Elektrophorese und Western-Blot wurden analog zu Beispiel 2 durchgeführt. Für die Immundetektion wurde abweichend von Beispiel 2 Hundeserum verwendet (1:1.000 in Blocklösung). Es wurde in diesem Fall alkalische Phosphatase gekoppelter Antikörper (1 mg/ml in TBS (50 mM TRIS/HCl, 150 mM NaCl, pH 7,5) eingesetzt. Die abschließende Entwicklung erfolgte dann wieder wie unter Beispiel 2 beschrieben.

### Beispiel 4

Elektrophorese und Western-Blot wurden analog zu Beispiel 2 durchgeführt. Für die Immundetektion wurde abweichend von Beispiel 2 Kälberserum verwendet (1:1.000 in Blocklösung). Es wurde in diesem Fall alkalische Phosphatase gekoppelter Antikörper (1 mg/ml in TBS (50 mM TRIS/HCl, 150 mM NaCl, pH 7,5) eingesetzt. Die abschließende Entwicklung erfolgte dann wieder wie unter Beispiel 2 beschrieben.

### Beispiel 5

Elektrophorese und Western-Blot wurden analog zu Beispiel 2 durchgeführt. Für die Immundetektion wurde abweichend von Beispiel 2 Schafserum verwendet (1:1.000 in Blocklösung). ES wurde in diesem Fall alkalische Phosphatase gekoppelter Antikörper (1 mg/ml in TBS (50 mM TRIS/HCl, 150 mM NaCl, pH 7,5) eingesetzt. Die abschließende Entwicklung erfolgte dann wieder wie unter Beispiel 2 beschrieben.

### Beispiel 6

Elektrophorese und Western-Blot wurden analog zu Beispiel 2 durchgeführt. Für die Immundetektion wurden abweichend von Beispiel 2 ausschließlich sekundäre, enzymgekoppelte Antikörper verwendet (Ziege-anti-Kaninchen-IgG gekoppelt mit alkalischer Phosphatase; 1:1.000 in Blocklösung). Die abschließende Entwicklung erfolgte dann wieder wie unter Beispiel 2 beschrieben.

### Beispiel 7

Elektrophorese und Western-Blot wurden analog zu Beispiel 2 durchgeführt. Für die Immundetektion wurden abweichend von Beispiel 2 ausschließlich sekundäre, enzymgekoppelte Antikörper verwendet (Ziege-anti-Kaninchen-IgG gekoppelt mit Meerrettich-Peroxidase; 1:1.000 in Blocklösung). Die abschließende Entwicklung erfolgte dann wieder wie unter Beispiel 2 beschrieben, wobei als Substratlösung Diaminobenzidin (0,6 mg/ml DAB in 100 mM Citrat/Azetatpuffer pH 6,0; 0,025% H2O2) eingesetzt wurde.

### Beispiel 8

Elektrophorese und Western-Blot wurden analog zu Beispiel 2 durchgeführt. Für die Immundetektion wurde abweichend von Beispiel 2 Pferderserum verwendet (1:1.000 in Blocklösung). Es wurde in diesem Fall alkalische Phosphatase gekoppelter Antikörper (1 mg/ml in TBS (50 mM TRIS/HCl, 150 mM NaCl, pH 7,5) eingesetzt. Die abschließende Entwicklung erfolgte dann wieder wie unter Beispiel 2 beschrieben.

### Beispiel 9

Elektrophorese und Western-Blot wurden analog zu Beispiel 2 durchgeführt. Für die Immundetektion wurde abweichend von Beispiel 2 monoklonales Mäuseserum verwendet (1:3.000 in 3% Blocklösung). Als sekundärer Antikörper wurde alkalische Phosphatase gekoppeltes Ziege-Anti-Kanninchen-IgG-Antiserum (1:10.000 in 3% Blocklösung) verwendet. Die abschließende Entwicklung erfolgte dann wieder wie unter Beispiel 2 beschrieben.

### Beispiel 10: SDS Polyacrylamidgelelektrophorese (SDS-PAGE)

Ein 1 mm dickes, 10 cm x 10 cm großes, 12,5%iges, diskontinuierliches SDS-Polyacrylamidgel wird vorbereitet und in eine Laufkammer eingesetzt. Die Proteinprobe und das Standardprotein bzw. die Mischung der Standardproteine werden in 35 µl Probenpuffer 5 Minuten aufgekocht, zentrifugiert und in die Laufkammern gefüllt. Die Trennung erfolgt bei einer Spannung von 10 V/cm, wobei die Elektrophorese-Apparatur wassergekühlt wird. Nach der Beendigung der Elektrophorese, üblicherweise 2 Stunden, werden die Glasplatten entfernt und das Gel für 45 Minuten in einer Coomassie-Blau Lösung geschwenkt. Danach wird das Gel in eine Entfärbelösung überführt, wodurch die Hintergrundfärbung abgeschwächt wird. Sowohl Standardproteine als auch Probenproteine werden gut sichtbar angefärbt. Die Ermittlung der Molekulargewichte kann durch Interpolation erfolgen.

### Beispiel 11: Western-Blot

Ein wie in Beispiel 1 erhaltenes Polyacrylamidgel wird vor oder nach der Färbung auf eine methanolgetränkte PVDF-Membran (Boehringer Mannheim) geblottet. Danach wird die Membran für eine Stunde in einer Blockierungs-Lösung geschwenkt und dreimal durch Schwenken in einer Waschlösung für 10 Minuten gewaschen. Anschließend wird mit dem primären Antikörper für eine Stunde inkubiert. Daran anschließend erfolgt die Inkubation mit einem sekundären peroxidasekonjugiertem Antikörper. Dieser Antikörper ist gegen den primären Antikörper gerichtet. Bei Zugabe von 0,05% Diaminobenzidin (DAB) und 0,005% Wasserstoffperoxid wird durch die Peroxidase ein unlöslicher Farbkomplex gebildet, was zu einer Anfärbung der Standardproteine und des zu analysierenden Proteins führt.

### Beispiel 12: Isoelektrische Fokussierung

Entsprechend den Angaben des Herstellers wird eine Minigelkasette zusammengesetzt und ein denaturierendes isoelektrisches Fokussiergel gegossen. Das Polyacrylamidgel enthält Ampholyte mit einem pH-Bereich von pH 3,5 bis 10. Nachdem das Gel polymerisiert ist, wird das Gel in die Gelkammer gestellt und die Anodenkammer mit 10 mM Phosphorsäure und die Kathodenkammer mit 20 mM NaOH gefällt. Die zu untersuchende Proteinprobe wird in einem denaturierenden Gel-Ladepuffer aufgenommen, 5 min bei 10.000 x g zentrifugiert und vom Überstand werden 20 µl in eine Probentasche aufgetragen. Parallel dazu werden die Standarproteine mit bekannten pI-Werten aufgetragen. Die Fokussierung wird zunächst mit 150 V für 30 min und dann mit 200 V für 2,5 Stunden durchgeführt, wobei das Gel durch Kühlung auf 20°C gehalten wird. Nach der Fokussierung kann das Gel mit einem Farbstoff wie z.B. Coomassie-blue oder Silber angefärbt werden und der pI der Probe durch Interpolation der pI-Werte der Standardproteine ermittelt werden.

## Patentansprüche

1. Standardprotein mit mindestens einer Antikörperbindungsdomäne aus Protein G, die mindestens einen nicht-konservativen Austausch einer Aminosäure aufweist.

2. Standardprotein nach Anspruch 1, dadurch gekennzeichnet, daß der mindestens eine nicht-konservative Austausch im ersten β-Faltblatt, im vierten β-Faltblatt oder in der α-Helix liegt.

3. Standardprotein nach Anspruch 1 oder 2, dadurch gekennzeichnet, daß der mindestens eine nicht-konservative Austausch in Position 3, 5, 26, 27, 29, 53 oder 56 liegt, insbesondere ausgewählt wird aus der Gruppe Y3R, Y3H, L5R, L5N, L5H, A26H, A26N, A26K, E27K, V29Y, T53R, E56T.

4. Standardprotein nach einem der Ansprüche 1 bis 3, dadurch gekennzeichnet, daß es mindestens zwei nicht-konservative Austausche aufweist.

5. Standardprotein gemäß mindestens einem der Ansprüche 1 bis 4, dadurch gekennzeichnet, daß das Standardprotein ein rekombinantes Protein ist, das aus Molekulargewichtsdomänen (1) und Bindungsdomänen (2) mit mindestens einer Bindungsstelle aufgebaut ist.

6. Standardprotein gemäß Anspruch 5, dadurch gekennzeichnet, daß die mindestens eine Bindungsstelle am C-Terminus oder am N-Terminus liegt.

7. Standardprotein gemäß mindestens einem der Ansprüche 1 bis 6, dadurch gekennzeichnet, daß die Antikörperbindungsdomäne nach einer unter denaturierenden Bedingungen durchgeführten elektrophoretischen Trennung funktional ist.

8. Mischung von Standardproteinen gemäß mindestens einem der Ansprüche 1 bis 7, dadurch gekennzeichnet, daß sich die Standardproteine in mindestens einer Eigenschaft insbesondere ihrem Molekulargewicht oder ihrem isoelektrischen Punkt unterscheiden.

9. Zusammenstellung enthaltend mehrere Standardproteine gemäß mindestens einem der Ansprüche 1 bis 7 in getrennten Gefäßen zur individuellen Herstellung einer Mischung gemäß Anspruch 8.

10. Expressionsvektor zur Expression des Standardproteins gemäß mindestens einem der Ansprüche 1 bis 7, dadurch gekennzeichnet, daß der Expressionsvektor mindestens eine Nukleotidsequenz für eine Molekulargewichtsdomäne (1) und mindestens eine Nukleotidsequenz für eine Bindungsdomäne (2) aufweist, die zusammen als Fusionsprotein exprimiert werden können.
